# EUROPEAN PATENT SPECIFICATION

(11) **EP 1 862 183 B1**
(45) Date of publication and mention of the grant of the patent: **03.11.2010**
(21) Application number: 06715523.4
(22) Date of filing: 10.03.2006
(51) Int. Cl.: A61K 47/12, A61K 9/107, A61K 9/127, A61K 31/337, A61K 47/10, A61K 47/34

(54) **KIT COMPRISING DRUG AND FAT EMULSION**
KIT ENTHALTEND EINEN WIRKSTOFF UND EINE FETTEMULSION
SYSTEME CONSTITUE D'UN MEDICAMENT ET D'UNE EMULSION

(30) Priority: 14.03.2005 JP 2005071640
(43) Date of publication of application: 05.12.2007
(73) Proprietor: OTSUKA PHARMACEUTICAL FACTORY, INC., Naruto-shi, Tokushima 772-8601 (JP)
(72) Inventor: TAKEDA, Koichi c/o OTSUKA PHARMACEUTICAL FACTORY, INC.,, Tokushima, 772-8601 (JP); MATSUDA, Kenji c/o OTSUKA PHARMACEUTICAL FACTORY, INC.,, Tokushima, 772-8601 (JP); TERAO, Toshimitsu c/o OTSUKA PHARMACEUTICAL FACTORY, INC.,, Tokushima, 772-8601 (JP); INOUE, Tadaaki c/o OTSUKA PHARMACEUTICAL FACTORY, INC.,, Tokushima, 772-8601 (JP); IMAGAWA, Takashi c/o OTSUKA PHARMACEUTICAL FACTORY, INC.,, Tokushima, 772-8601 (JP)
(74) Representative: Weller, Wolfgang
(86) International application number: PCT/JP2006/304753
(87) International publication number: WO 2006/098241

(56) References cited:
- EP-A1- 0 331 755
- WO-A1-96/02247
- WO-A2-00/53231
- WO-A2-02/080883
- JP-A- 10 502 921
- JP-A- 2002 080 399
- JP-A- 2002 538 224
- JP-A- 2003 500 368
- JP-A- 2004 504 342
- JP-A- 2004 524 368
- JP-A- 2005 504 070
- JP-B2- 2 688 235
- US-A- 6 046 230

## Description

### Technical Field

The present invention relates to a pharmaceutical composition which can permit a sparingly water-soluble drug substance to be solubilized or dispersed in a liquid medium, such as fat emulsions, liposomes, etc., to yield an injectable solution containing the sparingly water-soluble drug substance, wherein such solubilization or dispersion of the sparingly water-soluble drug substance can be completed for a shortened period of time.

### Background Art

In processing into a pharmaceutical preparation for injection a drug substance being chemically unstable in an aqueous solution or a sparingly water-soluble drug substance, there has been known a process for producing a pharmaceutical preparation for injection which comprises mixing such drug substance with a fat emulsion or liposome, etc. However, the process suffers from the defect that the resultant pharmaceutical preparation, when it contains the drug substance being unstable even in a fat emulsion, or the drug substance or additives are susceptible to undergo separation or precipitation with a length of time elapsed, cannot be stored for a prolonged period of time. As a means of eliminating such defect, there has been known a kit designed for preparation on the occasion of use which consists of two separately prepared components of a fat emulsion and a drug-substance composition for mixing on the occasion of use (refer to the Official Gazette of Japanese Patent No. 2688235), whereby a fat emulsion containing the drug-substance composition is prepared. The said drug-substance composition comprises a solvent, such as a liquid polyalkylene glycol, liquid alkylethanolamine, liquid polyhydric alcohol, etc. as well as an excipient, such as sugars or amino acids.

The above-mentioned kit is intended to overcome the defect that a fat emulsion containing a sparingly water-soluble drug cannot be stored for a prolonged period of time by allowing a sparingly water-soluble drug substance to be dissolved in a non-aqueous solvent, inclusive of such liquid polyalkylene glycols as polyethylene glycols, to thereby secure the long-term stability of said sparingly water-soluble drug substance, and also by admixing, on the clinical scene, a solution of the sparingly water-soluble drug substance in a polyalkylene glycol with the fat emulsion (fat dispersion). A solvent of such a liquid polyalkylene glycol as polyethylene glycol, when used solely, exhibits lowered surface-active property, and fails to permit such a drug substance as dissolved in such a liquid polyalkylene glycol as polyethylene glycol to be solubilized or dissolved in a fat emulsion for a shortened period of time. Because of this, for example, it is conceivable to add a non-ionic surfactant, such as Polysorbate, polyoxyethylene hardened castor oil, etc. to a solvent consisting of such a liquid polyalkylene glycol as polyethylene glycol, in advance of dissolving a drug substance in a liquid polyalkylene glycol solvent, and there are known an emulsion (refer to the Official Gazette of JP 2003-500368 A) which contains one or more therapeutic drugs being a water-insoluble or having relatively low water-solubility, one or more tocols, one or more auxiliary solvents selected from propylene glycol, glycerol, polyethylene glycol and polyvinylpyrrolidone, and one or more surfactants, a method (refer to the Official Gazette of JP Hei 8-127529 A) for stabilizing a fat emulsion which comprises mixing the fat emulsion with an injectable solution in the presence of a non-ionic surfactant, and the like. However, such non-ionic surfactants are known to constitute one of the causes for side effects or adverse reactions, such as onset of anaphylactic shock, and is problematic in terms of safety.

As a pharmaceutical preparation being free from any non-ionic surfactants, for example, there is known an emulsion (refer to the Official Gazette of JP Hei 10-502921 A) produced, for example, by dissolving paclitaxel in an alcohol solution, followed by addition of the equivalent volume of an oil and stirring to the transparent state, and removing the alcohol through rotary evaporation or evaporation under a nitrogen stream. In view of the fact that paclitaxel shows a reduced solubility in oils (refer to Adam, J.D., et al., Journal of the National Cancer Institute Monographs, 1993, vol. 15, p.141-147), however, it is concerned from a long-term standpoint that the above-described emulsion may be liable to cause problems in terms of stability, such as crystallization or crystal deposition.

Therefore, no pharmaceutical composition, which can permit a sparingly water-soluble drug substance to be solubilized or dispersed in a liquid medium, such as fat emulsions, cannot be said to exhibit adequate effects.

### Disclosure of the Invention

### Problems to be Solved by the Invention

The present invention has as its objective to provide a pharmaceutical composition which has a sparingly water-soluble drug substance solubilized or dispersed uniformly in a liquid medium and which is safe to humans, wherein the sparingly water-soluble drug substance as dissolved in such a base as polyethylene glycol can be diluted in a liquid medium, such as fat emulsion (fat dispersant), for a shortened period of mixing time.

### Means for Solving the Problems

The present inventors, with a specific view to solving the above-described problem, conducted repeatedly intensive research, and as a result, found that in diluting a sparingly water-soluble drug substance as dissolved in a solvent with a pharmaceutically allowable liquid medium, such as a fat emulsion (fat dispersant), etc., to provide a pharmaceutical preparation for administration, such as an injectable solution, the sparingly water-soluble drug substance, when processed into a pharmaceutical composition having the said drug substance dissolved in a solvent containing a base, such as polyethylene glycol, etc., and a fatty acid or its pharmaceutically allowable salt, is rapidly solubilized or dispersed in a liquid medium, and this consequently can permit the pharmaceutical composition containing the sparingly water-soluble drug substance to be mixed with a liquid medium, such as a fat emulsion, liposome, etc. for a shortened period of mixing time and also the sparingly water-soluble drug substance to be solubilized and dispersed uniformly in the liquid medium. Thus, the present inventors found that the pharmaceutical composition comprising (a) a base, (b) a sparingly water-soluble drug substance and (c) a fatty acid or its pharmaceutically allowable salt, when admixed with a liquid medium, such as a fat emulsion, liposome, etc., can surprisingly permit the sparingly water-soluble drug substance to be solubilized or dispersed in the liquid medium for an extremely shortened period of mixing time to thereby produce an excellent liquid pharmaceutical preparation for administration, such as an injectable solution, and the finding was followed by further research, leading to completion of the present invention.

Thus, the present invention relates to:
1. A kit of a fat emulsion containing a sparingly water-soluble drug substance to be prepared on the occasion of use, which comprises a pharmaceutical composition containing (a) a base being liquid at room temperature, (b) 1 to 40% (w/v) of a sparingly water-soluble drug substance and (c) 0.01 to 5% (w/v) of a fatty acid or its pharmaceutically allowable salt, and a fat emulsion containing 0.5 to 20% (w/v) of an emulsifier and 0.01 to 30% (w/v) of an oily component.
2. The kit of a fat emulsion containing a sparingly water-soluble drug substance to be prepared on the occasion of use according to Claim 1, wherein the base being liquid at room temperature is one or a mixture of not less than two selected from polyethylene glycol, propylene glycol and ethanol, and the sparingly water-soluble drug substance is paclitaxel or docetaxel, while the fatty acid or its pharmaceutically allowable salt is oleic acid or sodium oleate.
3. The kit of a fat emulsion containing a sparingly water-soluble drug substance to be prepared on the occasion of use according to Claim 1 or 2, wherein the amount of the fat emulsion against the pharmaceutical composition is in the range of 10- to 1200-fold by mass.
4. The kit of a fat emulsion containing a sparingly water-soluble drug substance to be prepared on the occasion of use according to Claim 1 or 2, wherein the amount of the fat emulsion against the pharmaceutical composition is in the range of 20- to 300-fold by mass.
5. A container having a plural number of compartments divided with a communicable partitioning means, **characterized in that** at least one of the compartments accommodates a pharmaceutical composition containing (a) a base which is one or a mixture of not less than two selected from a polyethylene glycol, propylene glycol and ethanol being liquid at room temperature, (b) 1 to 40% (w/v) of paclitaxel or docetaxel and (c) 0.01 to 5% (w/v) of oleic acid or sodium oleate and at least one of another accommodates a fat emulsion containing 0.5 to 20% (w/v) of an emulsifier and 0.01 to 30% (w/v) of an oily component at an amount of 10- to 1200-fold by mass against the aforementioned pharmaceutical composition.

### The Effect of the Invention

The pharmaceutical composition according to the present invention can be mixed with a liquid medium, such as a fat emulsion, liposome, etc. for a shortened period of time.

The pharmaceutical composition according to the present invention can be used to allow homogeneous solubilization or dispersion of a sparingly water-soluble drug substance in a liquid medium for an extremely shortened period of time.

Since the fatty acids or pharmaceutically allowable salts being usable in the present invention can be assured of safety to humans, the resultant solution having a sparingly water-soluble drug substance uniformly solubilized or dispersed in the liquid medium can be utilized as a pharmaceutical for administration in the liquid state, such as injectable solutions containing a sparingly water-soluble drug substance.

### The Best Mode for Carrying out the Invention

The pharmaceutical composition according to the present invention can be produced by mixing (a) a base, (b) a sparingly water-soluble drug substance and (c) a fatty acid or its pharmaceutically allowable salt by the known means (which means is not particularly limited, and includes, for example, stirring, shaking, etc.). Such mixing is can be performed at room temperature, and, if desired, may also be carried out under warming at ca. 40 to 70°C.

As used herein, the term "base" refers to whatever may be able to dissolve a sparingly water-soluble drug substance. Such base is preferably the alcohol compounds which exist in the liquid state at room temperature (ca. 1 to 30° C). The above-described alcohol compound may be exemplified by polyethylene glycols, ethanol or propylene glycol, etc., wherein the average molecular weight of such polyethylene glycols is not particularly limited and ranges preferably from ca. 100 to 800. Also, such polyethylene glycols can be used by suitably blending with polyethylene glycols with an average molecular weight of ca. 1,000 to 4,000 in such a range of proportions as may allow the resultant mixtures to remain liquid at room temperature. The base may be used in the pharmaceutical composition according to the present invention in such amounts as may permit the sparingly water-soluble drug substance and fatty acid or its pharmaceutically allowable salt to be contained at the individual concentrations to be described below.

The above-described polyethylene glycol may be used by admixing with miscellaneous alcohols (e.g., propylene glycol or ethanol), and their mixing mass ratio is not particularly limited.

As used herein, the term "sparingly water-soluble drug substance" refers to any drug substances which, being known to be utilizable in the field of pharmaceuticals, show a lowered degree of water solubility in relation to their effective doses, and specifically denotes any drug substances for which the term "slightly soluble", "very slightly soluble" or "practically insoluble or insoluble" is used for solubility described in Japanese Pharmacopeia 14^{th} Revised Edition, General Rules. In other words, said term refers to any drugs which, when 1g or 1mL thereof is dissolved in a solvent, require the volume of the solvent (the volume of water) of not less than 100mL (not more than 1% of the concentration), preferably not less than 1,000mL (not more than 0.1% of the concentration) and more preferably not less than 10,000mL (not more than 0.01% of the concentration).

The preferred sparingly water-soluble drug substance which is usable in the present invention includes taxines. Such taxines may be preferably exemplified by paclitaxel and docetaxel, while miscellaneous taxines include, for example, 7-epipaclitaxel, 10-desacetyl-paclitaxel, 10-desacetyl-7-epipaclitaxel, bacca- tin III, etc.

In addition, the sparingly water-soluble drug substance is not limited to the above-described taxanes, and includes, for example, immunosuppressive drugs, such as Cyclosporin A, etc., antifungal agents, such as amphotericin B, ketoconazole, etc., antibiotics, such as loxythromycin, clarithromycin, dirithromycin, etc., antineoplastic agents, such as camptothecine, etoposide, etc., antiemetic agents, such as domperidone, etc., vasodilators, such as dipyridamole, etc., cardiotonics, such as gitoxin, etc., drugs for peptic ulcer, such as sulpiride, etc. and the like.

The above-mentioned sparingly water-soluble drug substances may be used alone or as a drug mixture of not less than two thereof.

The concentration of the sparingly water-soluble drug substance in the pharmaceutical composition according to the present invention is not particularly limited, and there can be utilized any concentrations up to the saturated one, which is preferably ca. 1 to 40%(w/v) and more preferably ca. 2 to 15% (w/v).

The fatty acid which is usable in the present invention is straight-chain or branched, saturated or unsaturated fatty acids of ca. 8 to 22 carbon atoms. Such fatty acids may be exemplified by octanoic acid, capric acid, lauric acid, myristic acid, palmitic acid, stearic acid, arachidic acid, behenic acid, undecylenic acid, elaidic acid, linoleic acid, linolenic acid, arachidonic acid, etc.

The pharmaceutically allowable salts of the fatty acid include, for example, salts with alkali metals (e.g. potassium, sodium, lithium, etc.), with the sodium salts being preferable.

The above-mentioned fatty acids or pharmaceutically allowable salts thereof, among others, are preferably oleic acid or sodium oleate.

The above-described fatty acids or pharmaceutically allowable salts thereof may be used singly or concomitantly in more than two kinds thereof.

The concentration of the above-described fatty acid or pharmaceutically allowable salt thereof in the pharmaceutical composition according to the present invention is ca. 0.01 to 5% (w/v), preferably ca. 0.1 to 5% (w/v).

The mass ratio of the sparingly water-soluble drug substance to the fatty acids or pharmaceutically allowable salts thereof is in the range of ca. 2.5 to 1,250 parts by mass against part by mass of the fatty acids or pharmaceutically allowable salts thereof, preferably ca. 2.5 to 125 parts by mass.

"The pharmaceutically allowable liquid medium" in the present invention is not particularly specified, only if it is any pharmaceutically allowable liquid media, and may be exemplified by a fat emulsion containing an emulsifier and an oily component, distilled water for injection, physiologic saline, glucose solution or liposome, etc., with the fat emulsion or liposome being preferable.

When such liquid medium is a fat emulsion containing an emulsifier and oily ingredient, the usable emulsifier may be exemplified by phospholipids. As the phospholipid, use can be made of both natural and synthetic phospholipids. The natural phospholipid includes, for example, lecithins, such as yolk lecithin, purified yolk phosphatidylcholine, soybean lecithin, purified soybean phosphatidylcholine, etc. and partially or wholly hydrogenated yolk lecithin, hydrogenated purified yolk phosphatidylcholine, hydrogenated soybean lecithin, hydrogenated purified soybean phosphatidylcholine, etc., but is not limited thereto. The synthetic phospholipids may be exemplified by (1) derivatives of phosphatidylcholine by subjecting its acyl group to chemical conversion, such as dilauroylphosphatidylcholine, dimyristoylphosphatidylcholine, dipalmitoylphosphatidylcholine, distearoylphosphatidylcholine, dioleoylphosphatidylcholine, dilinoleoylphosphatidylcholine, 1-palmitoyl-2-oleoyl-phosphatidylcholine, etc.; (2) phosphatidylethanolamine and phosphatidylethanolamines modified with polyalkylene glycols, such as L-a-phosphatidylethanolamine of an egg origin, L-a-phosphatidylethanolamine of a soybean origin, distearoylphosphatidylethanolamine-polyethylene glycol 5000, etc.; (3) phosphatidylglycerols, such as dioleoylphosphatidylglycerol, dilauroylphosphatidylglycerol, dipalmitoylphosphatidylglycerol, dimyristoylphosphatidylglycerol, distearoylphosphatidylglycerol, 1-palmitoyl-2-oleoyl-phosphatidylglycerol, etc.; (4) phosphatidic acids, such as dipalmitoylphosphatidic acid, dimyristoylphosphatidic acid, etc.; (5) phosphatidylinositol; and (6) phosphatidylserine, and the like, but are not limited thereto.

These phospholipids can be utilized singly or as a mixture of not less than two kinds thereof. Among others, the preferred phospholipid is yolk lecithin, soybean lecithin, purified yolk phosphatidylcholine and purified soybean phosphatidylcholine.

The oily component which can be used in the above-described fat emulsion may be any fats and oils, only if they are usable in the field of pharmaceuticals, and the plant oils and triglycerides are ordinarily preferable. Said plant oil may be exemplified by soybean oil, cotton seed oil, rapeseed oil, sesame oil, corn oil, peanut oil, safflower oil, olive oil, castor oil, coconut oil, perilla oil, etc. The triglyceride may be exemplified by middle-chain fatty acid triglycerides (e.g., Panacet (tradename; produced by Nippon Oils and Fats Co.), ODO (tradename; manufactured by Nisshin Oil CO.), Coconade MT (tradename; produced by Kao Co.)), chemically synthesized triglycerides (e.g., 2-linoleoyl-1,3-dioctanoylglycerol, 2-linoleoyl-1,3-didecanoylglycerol, etc.), but is not limited thereto. One or not less than two kinds selected from the above-mentioned plant oils and triglycerides are suitably used.

Furthermore, such fats and oils are not limited to the above-mentioned plant oils and triglycerides, and use can be made of one or more than two kinds of fats, such as animal oils, mineral oils, synthetic oils, essential oils, etc. In addition, such animal oils, etc. can be used as a mixture with above-mentioned plant oils and/or triglycerides.

The procedure for preparing the above-described fat emulsion can be carried out, for example, by emulsifying an oily component in an aqueous medium with an emulsifier. The above-described emulsification can be effected by the per se known procedure, such as the procedure which involves adding water for injection to a mixture of an oil and fat with a phospholipid to effect crude emulsification, followed by fine emulsification (fundamental, intrinsic emulsification) with use of a suitable high-pressure emulsifying machine.

The above-mentioned crude emulsification can be effected, for example, by use of a homogenizer, etc. and may be performed under a nitrogen stream and/or under warming (e.g. at room temperature to ca. 80°C), if desired.

The fine emulsification is desirably effected, for example, by use of a high-pressure homogenizer, etc. to the mean particle size of not more than ca. 10µm, preferably not more than ca. 5µm, more preferably not more than ca. 1µm, further more preferably not more than ca. 0. 5pm, and particularly preferably not more than ca. 0.3pm. The fine emulsification is effected at an emulsification temperature of room temperature to ca. 100° C, preferably ca. 40 to 80° C and at an emulsification pressure of ca. 400 to 800kg/cm², preferably ca. 500 to 600kg/cm². The fine emulsification can be performed under a nitrogen stream, if desired.

Referring to the concentrations of the oily component and emulsifier which constitute the above-described liquid medium, these are not particularly limited. The oily component may be present at concentrations in the range of ca. 0.01 to 30% (w/v), preferably ca. 0.1 to 20% (w/v), and more preferably ca. 0.1 to 10% (w/v), while the emulsifier may be present at concentrations in the range of ca. 0.5 to 20% (w/v), preferably ca. 0.5 to 15% (w/v), and more preferably ca. 2 to 15% (w/v).

The above-described fat emulsion can be incorporated with a stabilizer. As the stabilizer, there can be utilized, among the above-mentioned phospholipids, (a) phospholipid derivatives of polyalkylene-glycol modified phosphatidylethanolamines, whose remaining hydroxyl moieties of glycerol are esterified with straight-chain or branched saturated or unsaturated fatty acids of 10 to 22 carbon atoms, preferably straight-chain or branched saturated or unsaturated fatty acids of 14 to 18 carbon atoms, (b) phosphatidylglycerol, (c) phosphatidic acid, (d) phosphatidylinositol and (e) phosphatidylserine. When such phospholipids are used as a stabilizer for the above-described liquid medium, such stabilizer itself can be utilized as a phospholipid to be used forpreparationof theliquidmedium. Further, other phospholipid may be selected for use as the phospholipid.

In addition to the above-described phospholipids, usable as a stabilizer are straight-chain or branched saturated or unsaturated fatty acids of 10 to 22 carbon atoms, preferably straight-chain or branched saturated or unsaturated fatty acids of 10 to 20 carbon atoms (e.g., lauric acid, myristic acid, palmitic acid, stearic acid, oleic acid, linoleic acid, a-linolenic acid, etc.) or salts thereof (e.g., sodium salts, potassium salts, etc.). Such stabilizers can be used solely or concomitantly as a mixture of not less than two thereof.

In cases where the liquid medium is a liposome, any emulsifiers can find advantageous application in the liposome, only if they are the emulsifiers in the above-described fat emulsions. Preferable emulsifiers are yolk lecithin, soybean lecithin, purified yolk phosphatidylcholine and purified soybean phosphatidylcholine. Such emulsifiers are preferably used in proportions of ca. 0.5 to 20% (w/v). Such liposomes can similarly be incorporated with the stabilizers which are used in the above-described fat emulsions.

In order to stabilize physically or chemically the liposome membrane and also regulate the membrane fluidity, additionally, there may be added cholesterol, cholesterol esters (e.g., cholesterol palmitate, cholesterol stearate, cholesterol oleate, etc.), polyglycerol fatty acid esters (e.g., diglyceryl monostearate, diglyceryl dioleate, hexaglycerylmonomyristate, etc.) and the like. The formulation amount of such membrane additives is not particularly limited but preferably in proportions of ca. 0.1 to 20% by mass against total oil and fat forming the liposome.

Liposomes can be prepared by emulsifying an emulsifier in an aqueous medium (e.g., water for injection, purified water, physiologic saline, etc.). The emulsification procedure is not particularly limited, and the known procedure can be employed. For example, there can be adopted a procedure which involves adding water for injection to a mixture comprising a phospholipid, stabilizer, etc., followed by crude emulsification, and effecting fine emulsification (final emulsification) by use of an appropriate high-pressure emulsifying equipment, extruder, etc.

The above-described liquid medium, such as a fat emulsion, liposome, etc., can be formulated with miscellaneous, pharmaceutically allowable additives, if desired. The additives include, for example, the known antioxidants, antimicrobial agents, pH adjusting agents, isotonic agents, etc., which can be formulated with the above-described kind of liquid media designed for use through injection. The antioxidant can be exemplified by sodium metabisulfite (which also acts as an antimicrobial agents), sodium sulfite, sodium bisulfite, potassium metabisulfite, potassium sulfite, a-tocopherol, etc. The antimicrobial agent includes, for example, sodium caprylate, methyl benzoate, sodium metabisulfite (which also acts as an antioxidant), sodium edetate, etc. As the pH adjusting agent, for example, use can be made of sodium hydroxide, hydrochloric acid, etc., while as the isotonic agent, for example, there may be used glycerol; sugars, such as glucose, fructose, maltose, etc.; sugar alcohols, such as sorbitol, xylitol, etc., and the like. The oil-soluble additives out of these can be used by admixing the same in advance with fats and oils constituting the above-mentioned liquid media, while the water-soluble additives can be utilized by dissolving the same in advance in a water-soluble solvent (e.g., water for injection, purified water, etc.) or incorporating the same into the aqueous phase of the resultant emulsion. The addition or formulation ratios of such additives are self-evident to a person of ordinary skill in the art, and do not make difference from the conventionally known ones. Also, cyclodextrins can be added to, and formulated with the liquid media, if desired.

The pharmaceutical composition according to the present invention can be mixed with the above-described pharmaceutically allowable liquid medium to allow a sparingly water-soluble drug substance to be readily solubilized or dispersed in the said liquid medium. As used in the present invention, the term "solubilization" refers to dissolution in the liquid medium of a sparingly water-soluble drug substance, while the term "dispersion" refers to fine and homogeneous suspension or emulsification of a sparingly water-soluble drug substance. Fine particles of a sparingly water-soluble drug substance in the state of dispersion, on the occasion of utilization as an injectable solution, preferably shows a particle size of not more than ca. 150 µm, and not more than ca. 7 µm in the case of application through intravascular injection.

The mixing amount of the above-described liquid medium against the pharmaceutical composition according to the present invention is not particularly limited, only if it can permit the sparingly water-soluble drug substance contained in the pharmaceutical composition to be solubilized or dispersed in the liquid medium, and may be in the range of ca. 10 to 1200 parts by mass of the liquid medium per part by mass of the pharmaceutical composition, preferably ca. 20 to 300 parts by mass, more preferably ca. 25 to 160 parts by mass.

The means for mixing the pharmaceutical composition according to the present invention with the above-described liquid medium is not particularly limited, and may include, for example, a procedure which involves conventional dissolution for mixing (e.g., irrigation, injection, etc.), followed by thorough shaking with a hand or shaking with use of a vortex shaker, etc., and the like. The shaking time varies depending upon the liquid volume, and is within ca. 2 min., preferably in the region of ca. 10 sec. to 1 min.

The pharmaceutical composition according to the present invention, when it is contained in at least one compartment in a container having a plural number of compartments divided by communicable partitioning means, with the above-described liquid medium being contained in at least one of the others, can be easily mixed aseptically with the liquid medium by bringing the divided compartments into communication through elimination of such partitioning means.

The present invention relates to a container which has the pharmaceutical composition according to the present invention and the liquid medium contained therein. The container is **characterized in that** said container has a plural number of compartments having at least two compartments which are divided by communicable partitioning means.

In at least one of the said plural number of compartments, there is accommodated the pharmaceutical composition according to the present invention. The pharmaceutical composition according to the present invention contains preferably ca. 1 to 40% (w/v) of paclitaxel or docetaxel, as well as a fatty acid and base. The fatty acid and base include, for example, those as mentioned above. At least one another of the said plural number of compartments accommodates a fat emulsion containing ca. 0.5 to 20% (w/v) of an emulsifier and ca. 0.01 to 30% (w/v) of an oily component, wherein the emulsifier and oily component may be exemplified by the ones as mentioned above.

The container, which contains the pharmaceutical composition and liquid medium, may be any containers, if they are made of the materials of construction being applicable as a container for medicinal uses, and there may be usable, for example, glass containers, plastic containers, etc. Such containers, on the occasion of manufacture or during storage, may be able to keep in the isolated state a plural number of compartments which are divided by communicable partitioning means. The communicable partitioning means may preferably be exemplified by weak seals which can be peeled off, and in addition to such weak seals, may be a container with a hole formed therethrough on the partition being provided among a plural number of compartments, which hole is designed to be closed during storage but to be opened through breaking of the closure on the occasion of use to thereby allow the plural number of compartments to communicate to one another. The preferred container of the present invention includes, for example, those as described in the Official Gazettes of JP No. 3079403, JP No. Hei 2-4671 A, JUM No. Hei 5-5138 A, JP Nos. Sho 63-309263 A and Sho 63-20550 A, etc. and the like.

The container according to the present invention can permit the partitioning section to be peeled off or demolished on the occasion of use to bring the container's inside into one-piece compartment, thereby facilitating the pharmaceutical composition according to the present invention and the liquid medium to be mixed. Also, the container according to the present invention, which is designed to merely allow each of the partitioning sections for the compartments to be peeled off or demolished on the occasion of use, can keep its inside tightly closed and permit the pharmaceutical composition according to the present invention and the liquid medium to be mixed aseptically.

In addition, the present invention relates to a kit of the pharmaceutical preparation for administration in the liquid state to be prepared on the occasion of use, which kit comprises the pharmaceutical composition according to the present invention and the liquid medium, for example, a kit of a fat emulsion containing sparingly water-soluble drug substance to be prepared on the occasion of use which kit comprises the pharmaceutical composition according to the present invention and the fat emulsion. The fat emulsion to be used in said kit can be suitably exemplified by a fat emulsion containing ca. 05 to 20% (w/v) of an emulsifier and ca. 0.01 to 30% (w/v) of an oily ingredient. The kit of a fat emulsion containing a sparingly water-soluble drug substance to be prepared on the occasion of use according to the present invention can be used by the procedure to be described below.

On the occasion of use, for example, a fat emulsion is injected for thorough mixing into a container having the pharmaceutical composition according to the present invention enclosed therein from a container enclosing the fat emulsion, or a pharmaceutical composition is injected for thorough mixing into a container enclosing a fat emulsion from a container enclosing the pharmaceutical composition according to the present invention.

In the above-described containers or kits, it is preferable to use the fat emulsion containing a sparingly water-soluble drug substance as prepared within the period of time during which the sparingly water-soluble drug substance remains stable in the fat emulsion from the standpoint of pharmaceutical preparation.

Also, a liposome can be used in place of the fat emulsion to render the above-described kit into a kit of a liposome containing a sparingly water-soluble drug substance.

To be described below are examples to illustrate the present invention in detail, but the present invention is not understood to be limited thereto. The abbreviations as used in the examples are as follows:

| | |
|---|---|
| PTL: | Paclitaxel |
| PEG: | Polyethylene glycol 400 |
| EtOH: | Ethanol |
| PG: | Propylene glycol |
| DSPG: | Distearoylphosphatidylglycerol |

The drug substances and compounds as used in the examples are as follows:
Paclitaxel (produced by Wako Pure Chemicals Co.)
Polyethylene glycol 400 (produced by Wako Pure Chemicals Co.)
Ethanol (produced by Wako Pure Chemicals Co.)
Propylene glycol (produced by Wako Pure Chemicals Co.) Oleic acid (produced by Aldrich Co.)
Soybean oil (purified soybean oil: produced by Nisshin Oil Manufacturing Co.)
Yolk lecithin (purified yolk lecithin: produced by QP Co.) DSPG (produced by Nippon Oils and Fats Co.)

### Example 1:

The ingredients as listed below in Table 1 were mixed for dissolution, and the resultant solution was filtered through filter (produced by PALL CO.) with a pore size of 0.2µm made of a polyether sulfone membrane, and filled in the 5mL portion into 5-mL glass vials, followed by tightly sealing to produce Pharmaceutical Composition 1.

**Table 1:**

| Pharmaceutical Composition 1 | |
|---|---|
| Ingredient | Formulated amount per 100mL |
| PTL | 12.5g |
| Oleic acid | 1g |
| PEG | Appropriate amount |

The ingredients as described in Table 2 except glycerol were mixed, and glycerol as dissolved in water for injection was added to the resultant mixture to the final concentration of 2. 21w/v%, followed by crude emulsification with warming under a nitrogen stream by use of a polytron homogenizer (manufactured by KINEMATICA. Co.) at 25000 rpm for 10 min.

Then, the resultant crude emulsion was subjected to fine emulsification to the mean particle size of not more than 0.3µm under a nitrogen stream by use of a high-pressure homogenizer (manufactured by APV Co.) at the emulsification temperature of 60°C and at the emulsification pressure of 550kg/cm². The resultant emulsion was adjusted to the specifically determined pH value with sodium hydroxide or hydrochloric acid, and filled in the 15.5mL portion into 20-mL glass vials, followed by tightly closing and high-pressure steam (at 121°C for 12 min) sterilization to prepare Liquid Medium 1 (fat emulsion).

**Table 2:**

| Liquid Medium 1 | |
|---|---|
| Ingredient | Formulated amount per 100mL |
| Soybean oil | 0.5g |
| Yolk lecithin | 3g |
| DSPG | 0.5g |
| Glycerol | 2.21g |
| Water for injection | Appropriate amount |

### Example 2

The ingredients as described below in Table 3 were mixed for dissolution, and the resultant solution was subjected to filtration, filling into vials and tight closing in the same manner as described in Example 1 for Pharmaceutical Composition 1 to prepare Pharmaceutical Composition 2. The same liquid medium (Liquid Medium 1) as described in Example 1 was prepared and used.

**Table 3:**

| Pharmaceutical Composition 2 | |
|---|---|
| Ingredient | Formulated amount per 100mL |
| PTL | 12.5g |
| Oleic acid | 0.1g |
| PEG | Appropriate amount |

### Example 3

The ingredients as described below in Table 4 were mixed for dissolution, and the resultant solution was subjected to filtration, filling into vials and tight closing in the same manner as described in Example 1 for Pharmaceutical Composition 1 to prepare Pharmaceutical Composition 3. The same liquid medium (Liquid Medium 1) as described in Example 1 was prepared and used.

**Table 4:**

| Pharmaceutical Composition 3 | |
|---|---|
| Ingredient | Formulated amount per 100mL |
| PTL | 12.5g |
| Sodium oleate | 0.1g |
| PEG | Appropriate amount |

### Example 4

The ingredients as described below in Table 5 were mixed for dissolution, and the resultant solution was subjected to filtration, filling into vials and tight closing in the same manner as described in Example 1 for Pharmaceutical Composition 1 to prepare Pharmaceutical Composition 4. The same liquid medium (Liquid Medium 1) as described in Example 1 was prepared and used.

**Table 5:**

| Pharmaceutical Composition 4 | |
|---|---|
| Ingredient | Formulated amount per 100mL |
| PTL | 12.5g |
| Oleic acid | 5g |
| PEG | Appropriate amount |

### Example 5

The ingredients as described below in Table 6 were mixed for dissolution, and the resultant solution was subjected to filtration, filling into vials and tight closing in the same manner as described in Example 1 for Pharmaceutical Composition 1 to prepare Pharmaceutical Composition 5. The same liquid medium (Liquid Medium 1) as described in Example 1 was prepared and used.

**Table 6:**

| Pharmaceutical Composition 5 | |
|---|---|
| Ingredient | Formulated amount per 100mL |
| PTL | 12.5g |
| Oleic acid | 5g |
| PEG | Appropriate amount |

### Example 6

The ingredients as described below in Table 7 were mixed for dissolution, and the resultant solution was subjected to filtration, filling into vials and tight closing in the same manner as described in Example 1 for Pharmaceutical Composition 1 to prepare Pharmaceutical Composition 6. The same liquid medium (Liquid Medium 1) as described in Example 1 was prepared and used.

**Table 7:**

| Pharmaceutical Composition 6 | |
|---|---|
| Ingredient | Formulated amount per 100mL |
| PTL | 3g |
| Oleic acid | 0.2g |
| EtOH | Appropriate amount |

### Example 7

The ingredients as described below in Table 8 were mixed for dissolution, and the resultant solution was subjected to filtration, filling into vials and tight closing in the same manner as described in Example 1 for Pharmaceutical Composition 1 to prepare Pharmaceutical Composition 7. The same liquid medium (Liquid Medium 1) as described in Example 1 was prepared and used.

**Table 8:**

| Pharmaceutical Composition 7 | |
|---|---|
| Ingredient | Formulated amount per 100mL |
| PTL | 10g |
| Oleic acid | 1g |
| PEG:PG=1:1 | Appropriate amount |

### Example 8

The same pharmaceutical composition as described in Example 1 for Pharmaceutical Composition 1 was prepared, while as a liquid medium, the ingredients as described below in Table 9 were treated in the same manner as described in Example 1 for Liquid Medium 1 to prepare Liquid Medium 2 (liposome).

**Table 9:**

| Liquid Medium 2 | |
|---|---|
| Ingredient | Formulated amount per 100mL |
| Yolk lecithin | 4g |
| Glycerol | 2.21g |
| Water for injection | Appropriate amount |

### Comparative Example 1

The ingredients as described below in Table 10 were mixed for dissolution, and the resultant solution was subjected to filtration, filling into vials and tight closing in the same manner as described in Example 1 for Pharmaceutical Composition 1 to prepare Pharmaceutical Composition 8. The same liquid medium (Liquid Medium 1) as described in Example 1 was prepared and used.

**Table 10:**

| Pharmaceutical Composition 8 | |
|---|---|
| Ingredient | Formulated amount per 100mL |
| PTL | 12.5g |
| PEG | Appropriate amount |

### Comparative Example 2

The same pharmaceutical composition (Pharmaceutical Composition 8) as described in Comparative Example 1 was prepared, while the ingredients as described below in Table 11 were treated in the same manner as described in Example 1 for Liquid Medium 1 to prepare Liquid Medium 3 (fat emulsion).

**Table 11:**

| Liquid Medium 3 | |
|---|---|
| Ingredient | Formulated amount per 100mL |
| Oleic acid | 0.0064g |
| Soybean oil | 0.5g |
| Yolk lecithin | 3g |
| DSPG | 0.5g |
| Glycerol | 2.21g |
| Water for injection | Appropriate amount |

### Comparative Example 3

The ingredients as described below in Table 12 were mixed for dissolution, and the resultant solution was subjected to filtration, filling into vials and tight closing in the same manner as described in Example 1 for Pharmaceutical Composition 1 to prepare Pharmaceutical Composition 9. The same liquid medium (Liquid Medium 1) as described in Example 1 was prepared and used.

**Table 12:**

| Pharmaceutical Composition 9 | |
|---|---|
| Ingredient | Formulated amount per 100mL |
| PTL | 3g |
| EtOH | Appropriate amount |

### Comparative Example 4

The ingredients as described below in Table 13 were mixed for dissolution, and the resultant solution was subjected to filtration, filling into vials and tight closing in the same manner as described in Example 1 for Pharmaceutical Composition 1 to prepare Pharmaceutical Composition 10. The same liquid medium (Liquid Medium 1) as described in Example 1 was prepared and used.

**Table 13:**

| Pharmaceutical Composition 10 | |
|---|---|
| Ingredient | Formulated amount per 100mL |
| PTL | 10g |
| PEG:PG=1:1 | Appropriate amount |

### Comparative Example 5

There were prepared the same pharmaceutical composition (Pharmaceutical Composition 8) and liquid medium (Liquid Medium 2) as described in Comparative Example 1 and Example 8, respectively.

### Test Example

The pharmaceutical compositions containing the amounts of Paclitaxel as indicated individually in Tables 14, 15 and 16 were added to 15.5mL each of the liquid media as obtained in Examples 1 to 8 and Comparative Examples 1 to 5, respectively, followed by stirring for mixing with a stirrer (at 600rpm), and the mixtures were subjected to time-course sampling to conduct microscopic observations for any insoluble matters (in Tables 14, 15 and 16, the symbols "+" and "-" denote the presence and absence of insoluble matters, respectively). The observations demonstrated that the pharmaceutical composition (Example 1) admixed with oleic acid required a shortened length of the mixing time to allow insoluble matters to disappear in the mixture of the pharmaceutical composition with the liquid medium, as compared with the pharmaceutical composition (Comparative Example 1) free from oleic acid. This shortened length of the mixing time was not able to be realized through admixture of the liquid medium with oleic acid (Comparative Example 2). Such mixing-time shortening effect during mixing of the pharmaceutical composition with a liquid medium, as produced by admixing the pharmaceutical composition with a fatty acid, was also observed with sodium oleate (Example 3). As for the base, when ethanol was used in place of polyethylene glycol 400, the pharmaceutical compositions, in which each base was admixed with a fatty acid, was found to produce the mixing-time shortening effect (Example 6).

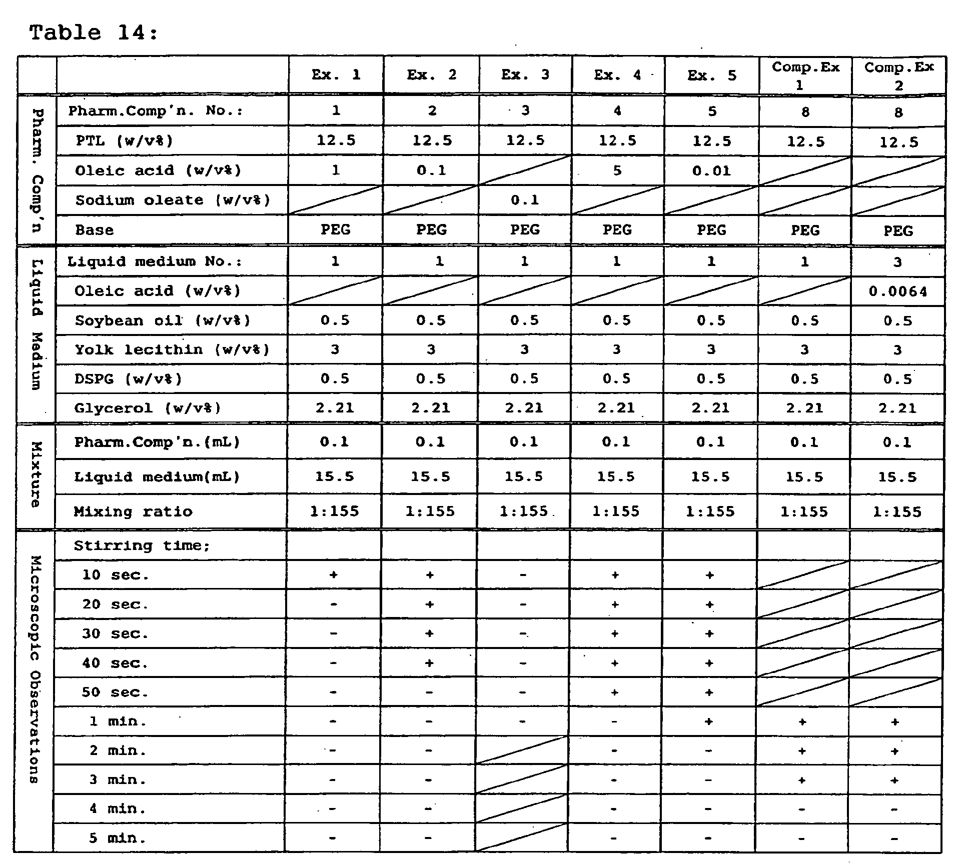

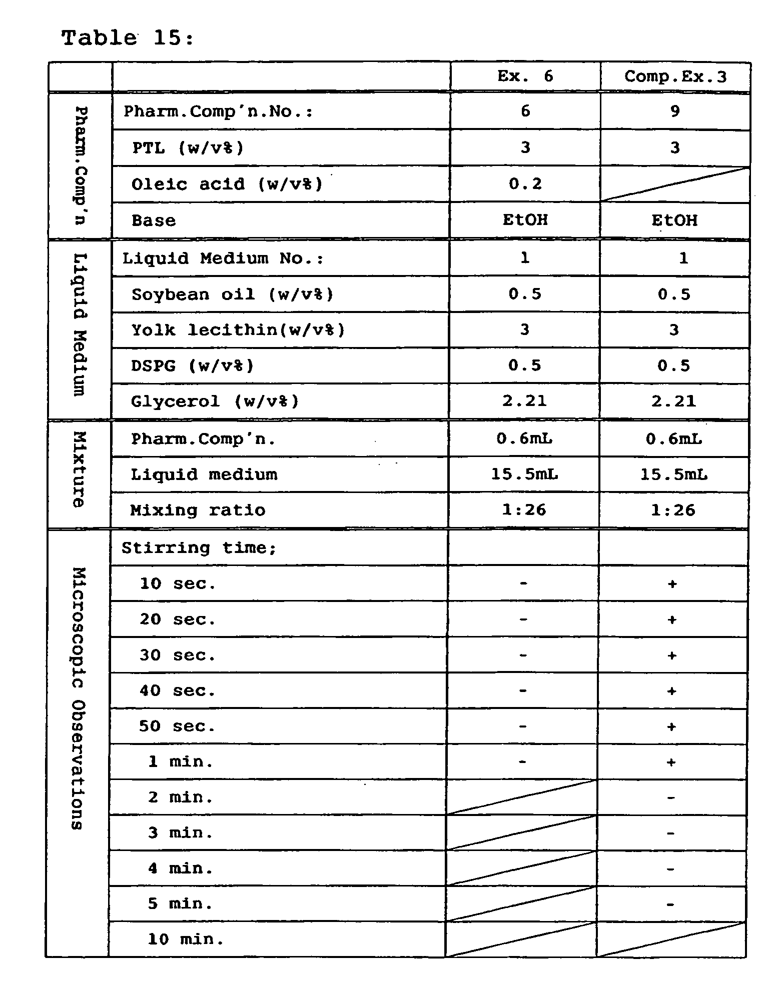

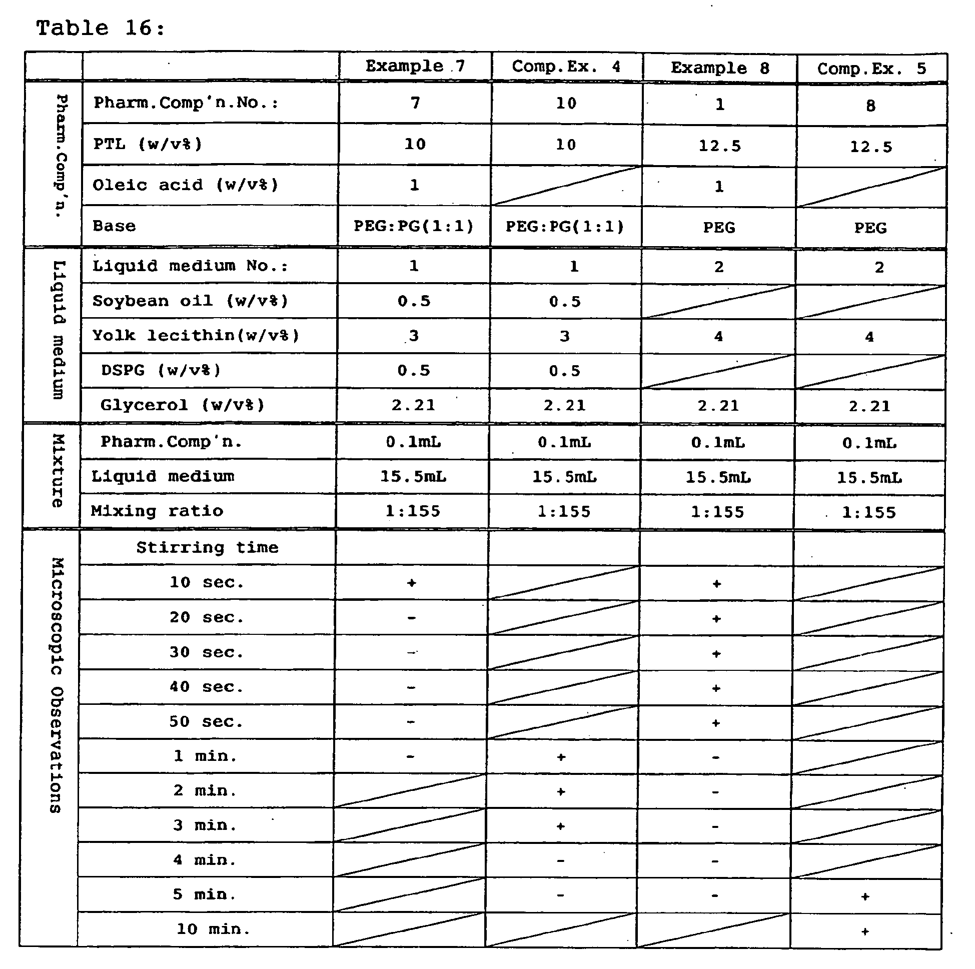

### Example 9

In a plastic-bag (ca.. 260pm thick) container made of a polyethylene having two compartments (Compartment A: 5mL in volume; Compartment B: 500mL in volume) with a communicable partition, 2mL of Pharmaceutical Composition 1 of Example 1 and 310mL of Liquid Medium 1 of Example 1 were filled in Compartments A and B, respectively, followed by closing and sterilization through hot-water showering.

The above-described container, on the occasion of utilization to a human, can be pressed from the outside to shift the partition between both of Compartments out of place and to make two compartments unified, thereby allowing the solutions in two compartments to be mixed easily.

### Industrial applicability

According to the present invention, in cases where a sparingly water-soluble drug substance as dissolved in such a base as polyethylene glycol is diluted with a fat emulsion (a dispersant for fats) to produce such a pharmaceutical preparation for administration, such as an injectable solution, a fatty acid or its pharmaceutically allowable salts can be added to the base, such as polyethylene glycol, to enable the sparingly water-soluble drug substance to be easily solubilized or dispersed in a pharmaceutically allowable liquid medium, and such advantageous action can shorten the length of time required to dissolve the sparingly water-soluble drug substance in the liquid medium. In addition, such fatty acids and their pharmaceutically allowable salts show an ensured degree of safety and can be used in practice.

## Claims

1. A kit of a fat emulsion containing a sparingly water-soluble drug substance which, when 1g or 1mL thereof is dissolved in a solvent, the volume of the solvent (the volume of water) of not less than 100mL is required to be prepared on the occasion of use, which comprises a pharmaceutical composition containing (a) a base being liquid at room temperature, (b) 1 to 40% (w/v) of a sparingly water-soluble drug substance and (c) 0.01 to 5% (w/v) of a fatty acid or its pharmaceutically allowable salt, and a fat emulsion containing 0.5 to 20% (w/v) of an emulsifier and 0.01 to 30% (w/v) of an oily component.

2. The kit of a fat emulsion containing a sparingly water-soluble drug substance to be prepared on the occasion of use according to Claim 1, wherein the base being liquid at room temperature is one or a mixture of not less than two selected from polyethylene glycol, propylene glycol and ethanol, and the sparingly water-soluble drug substance is paclitaxel or docetaxel, while the fatty acid or its pharmaceutically allowable salt is oleic acid or sodium oleate.

3. The kit of a fat emulsion containing a sparingly water-soluble drug substance to be prepared on the occasion of use according to Claim 1 or 2, wherein the amount of the fat emulsion against the pharmaceutical composition is in the range of 10- to 1200-fold by mass.

4. The kit of a fat emulsion containing a sparingly water-soluble drug substance to be prepared on the occasion of use according to Claim 1 or 2, wherein the amount of the fat emulsion against the pharmaceutical composition is in the range of 20- to 300-fold by mass.

5. A container having a plural number of compartments divided with a communicable partitioning means, **characterized in that** at least one of the compartments accommodates a pharmaceutical composition containing (a) a base which is one or a mixture of not less than two selected from a polyethylene glycol, propylene glycol and ethanol being liquid at room temperature, (b) 1 to 40% (w/v) of paclitaxel or docetaxel and (c) 0.01 to 5% (w/v) of oleic acid or sodium oleate and at least one of another accommodates a fat emulsion containing 0.5 to 20% (w/v) of an emulsifier and 0.01 to 30% (w/v) of an oily component at an amount of 10- to 1200-fold by mass against the aforementioned pharmaceutical composition.

## Patentansprüche

1. Kit für eine Fettemulsion, die eine wenig wasserlösliche Arzneimittelsubstanz enthält, die, wenn 1 g oder 1 ml davon in einem Lösungsmittel gelöst wird, ein Lösungsmittelvolumen (Wasservolumen) von nicht weniger als 100 ml benötigt, zur Zubereitung zum Zeitpunkt der Verwendung, das Folgendes aufweist, nämlich eine pharmazeutische Zusammensetzung, die Folgendes enthält, nämlich (a) eine bei Raumtemperatur flüssige Basis, (b) 1 bis 40 % (w/v) einer wenig wasserlöslichen Arzneimittelsubstanz und (c) 0,01 bis 5 % (w/v) einer Fettsäure oder ihres pharmazeutisch zulässigen Salzes, und eine Fettemulsion, die 0,5 bis 20 % (w/v) eines Emulgators und 0,01 bis 30 % (w/v) einer öligen Komponente aufweist.

2. Kit für eine Fettemulsion, die eine wenig wasserlösliche Arzneimittelsubstanz enthält, zur Zubereitung zum Zeitpunkt der Verwendung nach Anspruch 1, wobei die bei Raumtemperatur flüssige Basis eine oder eine Mischung von nicht weniger als zwei Substanzen ist, die ausgewählt sind aus Polyethylenglycol, Propylenglycol und Ethanol, und wobei die wenig wasserlösliche Arzneimittelsubstanz Paclitaxel oder Docetaxel ist, wobei die Fettsäure oder ihr pharmazeutisch zulässiges Salz Ölsäure oder Natriumoleat ist.

3. Kit für eine Fettemulsion, die eine wenig wasserlösliche Arzneimittelsubstanz aufweist, zur Zubereitung zum Zeitpunkt der Verwendung nach Anspruch 1 oder 2, wobei die Menge der Fettemulsion bezogen auf die pharmazeutische Zusammensetzung im Bereich des 10- bis 1200-fachen bezogen auf die Masse liegt.

4. Kit für eine Fettemulsion, die eine wenig wasserlösliche Arzneimittelsubstanz enthält, zur Zubereitung zum Zeitpunkt der Verwendung nach Anspruch 1 oder 2, wobei die Menge der Fettemulsion bezogen auf die pharmazeutische Zusammensetzung im Bereich des 20- bis 300-fachen bezogen auf die Masse liegt.

5. Ein Behälter mit einer Vielzahl von Abteilen, die durch untereinander in Verbindung bringbare Abteilungsmittel aufgeteilt sind, **dadurch gekennzeichnet, dass** zumindest eines der Abteile eine pharmazeutische Zusammensetzung aufnimmt, die Folgendes enthält, nämlich (a) eine Basis, die eine oder eine Mischung von nicht weniger als zwei Substanzen ist, die ausgewählt sind aus Polyethylenglycol, Propylenglycol und Ethanol, und die bei Raumtemperatur flüssig ist, (b) 1 bis 40 % (w/v) Paclitaxel oder Docetaxel und (c) 0,01 bis 5 % (w/v) Ölsäure oder Natriumoleat, und dass zumindest eines der weiteren, eine Fettemulsion, die Folgendes enthält, nämlich 0,5 bis 20 % (w/v) eines Emulgators und 0,01 bis 30 % (w/v) einer öligen Komponente in einer Menge des 10- bis 1200-fachen bezogen auf die Masse und bezogen auf die zuvor genannte pharmazeutische Zubereitung, aufnimmt.

## Revendications

1. Kit d'une émulsion grasse contenant une substance médicamenteuse à peine soluble dans l'eau qui, lorsque 1 g ou 1 ml de celle-ci est dissous dans un solvant, necessit un volume du solvant (le volume d'eau) de non moins de 100 ml, à préparér au moment de son utilisation, qui kit comprend une composition pharmaceutique qui contient (a) une base liquide à la température ambiante, (b) de 1 à 40 % (p/v) d'une substance médicamenteuse à peine soluble dans l'eau et (c) de 0,01 à 5 % (p/v) d'un acide gras ou de son sel pharmaceutiquement acceptable, et une émulsion grasse qui contient de 0,5 à 20 % (p/v) d'un émulsifiant et de 0,01 à 30 % (p/v) d'un composant huileux.

2. Kit d'une émulsion grasse contenant une substance médicamenteuse à peine soluble dans l'eau à préparer au moment de son utilisation selon la revendication 1, dans lequel la base liquide à la température ambiante est un élément ou un mélange d'au moins deux éléments choisi(s) parmi le polyéthylène glycol, le propylène glycol et l'éthanol, et la substance médicamenteuse à peine soluble dans l'eau est du paclitaxel ou du docetaxel, alors que l'acide gras ou son sel pharmaceutiquement acceptable est l'acide oléique ou l'oléate de sodium.

3. Kit d'une émulsion grasse contenant une substance médicamenteuse à peine soluble dans l'eau à préparer au moment de son utilisation selon la revendication 1 ou 2, dans lequel la quantité de l'émulsion grasse par rapport à la composition pharmaceutique se situe dans la plage de 10 à 1200 fois par masse.

4. Kit d'une émulsion grasse contenant une substance médicamenteuse à peine soluble dans l'eau à préparer au moment de son utilisation selon la revendication 1 ou 2, dans lequel la quantité de l'émulsion grasse par rapport à la composition pharmaceutique se situe dans la plage de 20 à 300 fois par masse.

5. Récipient comportant une pluralité de compartiments divisés avec un dispositif de cloisonnement pouvant communiquer, **caractérisé en qu'**au moins un des compartiments contient une composition pharmaceutique qui contient (a) une base qui est un élément ou un mélange d'au moins deux éléments choisi(s) parmi un polyéthylène glycol, un propylène glycol et l'éthanol et qui est liquide à la température ambiante, (b) de 1 à 40 % (p/v) de paclitaxel ou de docetaxel et (c) de 0,01 à 5 % (p/v) d'acide oléique ou d'oléate de sodium, et en ce qu'au moins un autre compartiment contient une émulsion grasse qui contient de 0,5 à 20 % (p/v) d'un émulsifiant et de 0,01 à 30 % (p/v) d'un composant huileux en une quantité de 10 à 1200 fois par masse par rapport à la composition pharmaceutique mentionnée ci-dessus.
